# EUROPEAN PATENT APPLICATION

(11) **EP 4 349 337 A1**
(43) Date of publication of application: **10.04.2024**
(21) Application number: 22811283.5
(22) Date of filing: 23.05.2022
(51) Int. Cl.: A61K 31/4745, A61K 9/127, A61K 47/04, A61K 47/24, A61K 47/28

(54) **ANTI-TUMOR AGENT**

(30) Priority: 24.05.2021 US 202163192366 P
(71) Applicant: FUJIFILM Corporation, Tokyo 106-8620 (JP)
(72) Inventor: SHIMOYAMA Susumu, Cambridge, Massachusetts 02142 (US); SUZUKI Keiko, Ashigarakami-gun, Kanagawa 258-8577 (JP); MORI Mikinaga, Ashigarakami-gun, Kanagawa 258-8577 (JP); MATSUMOTO Takeshi, Ashigarakami-gun, Kanagawa 258-8577 (JP); NAKAYAMA Shinji, Ashigarakami-gun, Kanagawa 258-8577 (JP); MOROHASHI Yasushi, Ashigarakami-gun, Kanagawa 258-8577 (JP); KIMURA Toshifumi, Ashigarakami-gun, Kanagawa 258-8577 (JP)
(74) Representative: Hoffmann Eitle
(86) International application number: PCT/JP2022/021103
(87) International publication number: WO 2022/250013

(57) **Abstract**

An object of the present invention is to provide an anti-tumor agent that exhibits a remarkably excellent anti-tumor effect. According to the present invention, there is provided an anti-tumor agent for curing cancer, the anti-tumor agent having a liposome which has an inner water phase and having an aqueous solution which is an outer water phase and disperses the liposome, in which the liposome encompasses topotecan or a salt thereof, a lipid constituting the liposome contains a lipid modified with polyethylene glycol, dihydrosphingomyelin, and cholesterol, the inner water phase contains an ammonium salt, and the topotecan or the salt thereof encompassed in the liposome is administered at a dose rate of 0.1 mg/m² body surface area to 10 mg/m² body surface area, in terms of topotecan per administration.

## Description

### Technical Field

The present invention relates to an anti-tumor agent in which a liposome encompasses topotecan or a salt thereof, where the anti-tumor agent is administered according to a specific amount and a specific schedule.

### Background Art

In chemotherapy, it is often studied that a drug is accumulated at a disease site such as cancer and exposed thereto over a long period of time by means of a liposome composition.

Patent Document 1 describes that a liposome composition containing a hydrophilic polymer-modified diacylphosphatidylethanolamine, dihydrosphingomyelin, and cholesterols as the constitutional components of the liposome membrane exhibits a high AUC.

Patent Document 2 discloses a liposome in which topotecan is encapsulated in a liposome containing sphingomyelin and cholesterol.

Patent Document 3 discloses a liposome in which topotecan is encapsulated in a liposome containing dihydrosphingomyelin and cholesterol.

Patent Document 4 discloses a liposomal camptothecin preparation adapted to enhance the stability of camptothecin, including (a) camptothecin encapsulated in a liposome, (b) first solution which is external to the liposome and has a pH of 4.5 or less than 4.5, and (c) second solution which is internal to the liposome. It is also disclosed that the liposome contains dihydrosphingomyelin and cholesterol.

Patent Document 5 discloses a liposome in which topotecan is encapsulated in the presence of ammonium sulfate in a liposome containing purified hydrogenated soybean phospholipid or sphingomyelin, cholesterol, and a hydrophilic polymer derivative lipid.

### Prior Art Documents

### Patent Documents

Document 1: WO2018/181963
Document 2: US7060828B2
Document 3: US7811602B2
Document 4: JP2008-519045A
Document 5: US6355268B2

### SUMMARY OF THE INVENTION

### Object to be solved by the invention

So far, there have been no reports on the results of the administration of liposomes encompassing topotecan or a salt thereof to cancer patients. As a result, it is not known what kind of blood kinetics the liposomes encompassing topotecan or a salt thereof exhibit. Even a person skilled in the art cannot estimate what dose rate makes it possible to achieve that liposomes encompassing topotecan or a salt thereof exhibit the drug efficacy of the gemcitabine or salt thereof to cancer patients unless actually administering them to cancer patients. In addition, the value of blood concentration of the liposomes encompassing topotecan or a salt thereof, required to exhibit an anti-tumor effect, has not been studied so far.

An object of the present invention is to provide an anti-tumor agent that exhibits a remarkably excellent anti-tumor effect.

### Means for solving the object

As a result of diligent studies to solve the above problems, the inventors of the present invention found that a particularly excellent anti-tumor effect can be obtained in a case of being administered at a specific dose rate and schedule, thereby completing the present invention.

That is, the present invention provides the following aspects.
[1] An anti-tumor agent for curing cancer, the anti-tumor agent comprising:
   a liposome which has an inner water phase; and
   an aqueous solution which is an outer water phase and disperses the liposome,
   in which the liposome encompasses topotecan or a salt thereof,
   a lipid constituting the liposome contains a lipid modified with polyethylene glycol, dihydrosphingomyelin, and cholesterol,
   the inner water phase contains an ammonium salt, and
   the topotecan or the salt thereof encompassed in the liposome is administered at a dose rate of 0.1 mg/m² body surface area to 10 mg/m² body surface area, in terms of topotecan per administration.
[2] The anti-tumor agent according to [1], in which the dihydrosphingomyelin is dihydrosphingomyelin having an alkyl group having 16 carbon atoms and an alkyl group having 18 carbon atoms.
[3] The anti-tumor agent according to [1] or [2], in which the ammonium salt contained in the inner water phase of the liposome contained in the anti-tumor agent is ammonium sulfate, and a molar ratio of sulfate ions contained in the inner water phase to the molar sum of the topotecan or the salt thereof contained in the anti-tumor agent is 0.36 or more in terms of topotecan.
[4] The anti-tumor agent according to any one of [1] to [3], in which a plurality of times of single administration is repeated every one week to eight weeks.
[5] The anti-tumor agent according to any one of [1] to [4], in which the cancer is a solid cancer.
[6] The anti-tumor agent according to [5], in which the solid cancer is at least one selected from breast cancer, uterine cancer, ovarian cancer, lung cancer, Merkel cell cancer, neuroendocrine tumor, or brain tumor.
[7] The anti-tumor agent according to any one of [1] to [6], in which the lipid modified with polyethylene glycol is diacylphosphatidylethanolamine modified with polyethylene glycol or methoxypolyethylene glycol.
[8] The anti-tumor agent according to any one of [1] to [7], in which a formulation ratio of the cholesterol to a total of the lipid constituting the liposome is 35% to 43% by mole.
[9] The anti-tumor agent according to any one of [1] to [8], in which a pH of the outer water phase is 5.5 to 8.5.
[10] The anti-tumor agent according to any one of [1] to [9], in which an administration route is an intravenous administration.
[11] The anti-tumor agent according to any one of [1] to [10], in which the anti-tumor agent is administered by infusion for 5 minutes to 360 minutes in a single administration.

[A] A treatment method for curing cancer, the treatment method comprising:
   a step of administering a composition,
   in which the anti-tumor agent contains;
   a liposome which has an inner water phase, and
   an aqueous solution which is an outer water phase and disperses the liposome,
   where the liposome encompasses topotecan or a salt thereof,
   a lipid constituting the liposome contains a lipid modified with polyethylene glycol, dihydrosphingomyelin, and cholesterol,
   the inner water phase contains an ammonium salt, and
   the topotecan or the salt thereof encompassed in the liposome is administered at a dose rate of 0.1 mg/m² body surface area to 10 mg/m² body surface area, in terms of topotecan per administration.
[B] An anti-tumor agent for using in curing of cancer, the anti-tumor agent comprising:
   a liposome which has an inner water phase; and
   an aqueous solution which is an outer water phase and disperses the liposome,
   in which the liposome encompasses topotecan or a salt thereof,
   a lipid constituting the liposome contains a lipid modified with polyethylene glycol, dihydrosphingomyelin, and cholesterol,
   the inner water phase contains an ammonium salt, and
   the topotecan or the salt thereof encompassed in the liposome is administered at a dose rate of 0.1 mg/m² body surface area to 10 mg/m² body surface area, in terms of topotecan per administration.
[C] A use of a composition for production of an anti-tumor agent for curing cancer,
   in which the composition contains;
   a liposome which has an inner water phase; and
   an aqueous solution which is an outer water phase and disperses the liposome,
   where the liposome encompasses topotecan or a salt thereof,
   a lipid constituting the liposome contains a lipid modified with polyethylene glycol, dihydrosphingomyelin, and cholesterol,
   the inner water phase contains an ammonium salt, and
   the topotecan or the salt thereof encompassed in the liposome is administered at a dose rate of 0.1 mg/m² body surface area to 10 mg/m² body surface area, in terms of topotecan per administration.

### Effect of the Invention

According to the anti-tumor agent according to the aspect of the present invention, an excellent anti-tumor effect is obtained, and the effect of reducing the volume of cancer is remarkable. In addition, as a result of the anti-tumor effect obtained from the anti-tumor agent according to the aspect of the present invention, it is expected that the extension of progression-free survival time and overall survival time, the improvement of QOL, and the like are achieved.

### BRIEF DESCRIPTION OF THE DRAWINGS

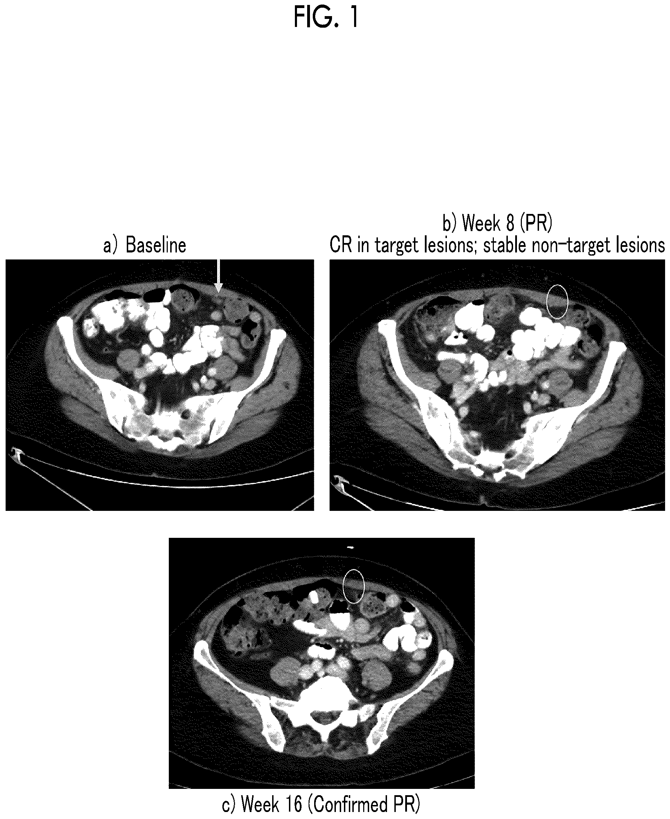

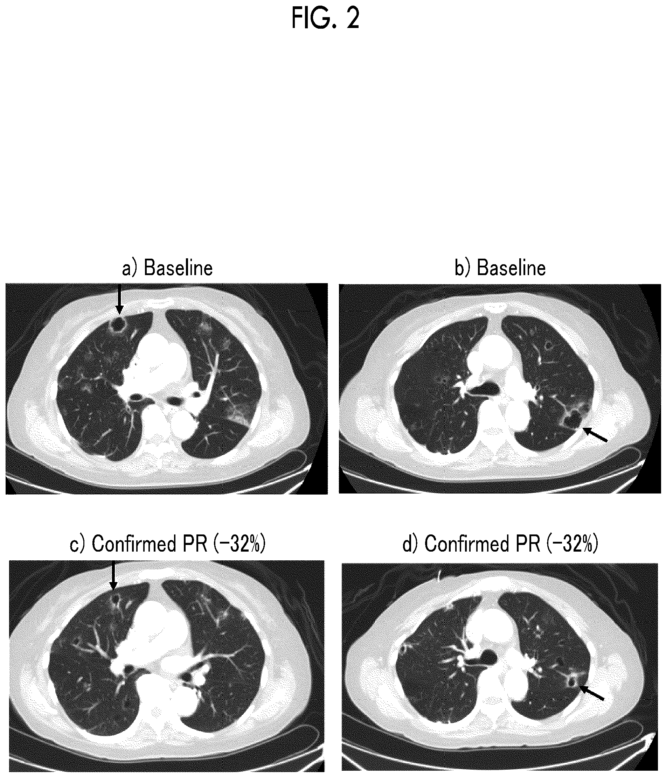

a of Fig. 1a to c of Fig, 1 show CT images in a case where a curing effect on an ovarian cancer patient 3 has been confirmed.
A of Fig. 2 to d of Fig. 2 show CT images in a case where a curing effect on cervical cancer patients has been confirmed.

### DESCRIPTION OF THE PREFERRED EMBODIMENTS

In the present invention, the range represented by "to" includes the values at both ends thereof unless otherwise specified.

In the present invention, the "tumor" is used synonymously with "malignant tumor" and "cancer". The "malignant tumor" means a tumor in which the morphology of tumor cells and the arrangement thereof are different from those of the normal cells from which the tumor cells are derived and which is invasive or metastatic.

The "treatment" means curing each disease.

The "subject" is a mammal such as a human, a mouse, a monkey, or a domestic animal requiring prevention or curing therefor, and preferably a human requiring prevention or curing therefor.

The "prevention" means the inhibition of the onset of a disease, the reduction of the risk of the onset of a disease, or the delay of the onset of a disease.

The "curing" means the amelioration or the suppression (the maintenance or delay) of the progression of a disease or state.

The "progression-free survival time" means the time during which cancer has not progressed and remained in a stable state during curing (after curing).

The "overall survival time" means the time during which a subject has survived from the allocation start date of therapy or curing start date in a clinical test.

Hereinafter, the present invention will be described in detail.

The present invention provides an anti-tumor agent for curing cancer, the anti-tumor agent having:
a liposome which has an inner water phase; and
an aqueous solution which is an outer water phase and disperses the liposome,
in which the liposome encompasses topotecan or a salt thereof,
a lipid constituting the liposome contains a lipid modified with polyethylene glycol, dihydrosphingomyelin, and cholesterol,
the inner water phase contains an ammonium salt, and
the topotecan or the salt thereof encompassed in the liposome is administered at a dose rate of 0.1 mg/m² body surface area to 10 mg/m² body surface area, in terms of topotecan per administration.

### (Liposome)

A liposome is a closed vesicle formed of a lipid bilayer membrane using a lipid, and an aqueous phase (an inner water phase) is included in the space of the closed vesicle. The inner water phase contains water. The liposome is generally present in a state of being dispersed in an aqueous solution (an outer water phase) outside the closed vesicle. The liposome may be a single lamella (which is also called a single-layer lamella or uni-lamella, where the bilayer membrane has a single layered structure) or may be a multi-layer lamella (which is also called a multi-lamella and has a structure of a large number of bilayer membranes, having an onion-like shape, where the individual layers are separated by an aqueous layer). However, in the present invention, a single lamellar liposome is preferable from the viewpoint of safety and stability in pharmaceutical use applications.

The form of the liposome is not particularly limited as long as the liposome is a liposome capable of encompassing a drug. The "encompassing" means taking a form in which a drug is contained in the inner water phase of the liposome. Examples thereof include a form in which a drug is enclosed in a closed space formed of a membrane and a form in which a drug is encompassed in the membrane itself, where a combination of these may be good.

In general, the average particle diameter of the liposome is 10 nm to 1,000 nm, preferably 20 nm to 500 nm, more preferably 30 nm to 300 nm, still more preferably 30 nm to 200 nm, even still more preferably 30 nm to 150 nm, and particularly preferably 50 to 150 nm. The liposome preferably has a spherical shape or a shape close thereto.

In a case of expecting the enhanced permeability and retention effect (the EPR effect), the diameter is preferably substantially 50 to 200 nm, the diameter is more preferably substantially 50 to 150 nm, and the diameter is still more preferably substantially 50 to 100 nm. The term "substantially" means that at least 75% of the number of liposomes is within the specified diameter range. The above-described "at least 75%" is more preferably at least 80% and still more preferably at least 90%.

It is noted that in the present invention, the "average particle diameter" means an average particle diameter (preferably, a cumulant average particle diameter) measured by using a dynamic light scattering method unless otherwise specified. The "average particle diameter" can be measured by using a device that can measure the average particle diameter according to a light scattering method.

The component constituting the lipid bilayer of the liposome is selected from lipids. The liposome in the present invention includes a hydrophilic polymer-modified diacylphosphatidylethanolamine, dihydrosphingomyelin, and cholesterol as the constitutional components of the liposome membrane.

The liposome according to the embodiment of the present invention contains dihydrosphingomyelin. The retention of the liposome in the blood can be improved by using dihydrosphingomyelin. In addition, it is possible to improve the partition properties of the liposome membrane and prevent the leakage of topotecan or a salt thereof.

Dihydrosphingomyelin generally has two long-chain alkyl groups in the molecule, and examples thereof include dihydrosphingomyelin having two long-chain alkyl groups respectively having 16 carbon atoms, dihydrosphingomyelin having a long-chain alkyl group having 16 carbon atoms and a long-chain alkyl group having 18 carbon atoms, and dihydrosphingomyelin having a long-chain alkyl group having 16 carbon atoms and a long-chain alkyl group having 20 to 24 carbon atoms.

From the viewpoint of preventing leakage of a drug from the liposome, it is preferable to use, as the dihydrosphingomyelin, the following compound having a long-chain alkyl group having 16 carbon atoms and a long-chain alkyl group having 18 carbon atoms. This is because the melting point becomes higher as the number of carbon atoms is larger, and therefore a liposome membrane having high partition properties can be formed.

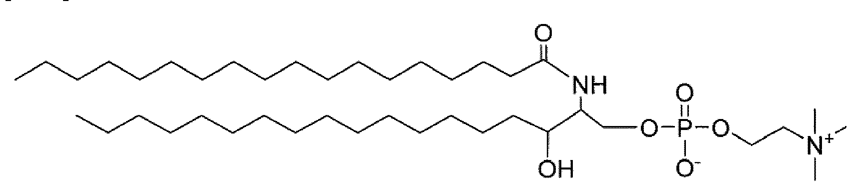

As the dihydrosphingomyelin, for example, dihydrosphingomyelin obtained by reducing naturally occurring sphingomyelin by a general method may be used, or dihydrosphingomyelin obtained by synthesis may be used.

Since most dihydrosphingomyelins derived from natural products such as chicken eggs generally have two long-chain alkyl groups respectively having 16 carbon atoms, it is preferable to use dihydrosphingomyelin obtained by chemical synthesis, from the viewpoint that dihydrosphingomyelin having a long-chain alkyl group having 16 carbon atoms and a long-chain alkyl group having 18 carbon atoms can be obtained with high purity.

The ratio of dihydrosphingomyelin in the total lipids constituting the liposome is preferably 30% to 80% by mole, more preferably 40% to 70% by mole, and still more preferably 50% to 60% by mole.

The liposome according to the embodiment of the present invention contains a lipid modified with polyethylene glycol (hereinafter, referred to as a PEG-modified lipid). It is noted that as the polyethylene glycol, a derivative thereof can also be used, and examples the derivative thereof include methoxypolyethylene glycol. Hereinafter, in a case of being referred to as PEG modification, the PEG modification also applies to a derivative of polyethylene glycol.

Examples of the PEG-modified lipid include a PEG-modified phospholipid, a PEG-modified monoglyceride, a PEG-modified diglyceride, a PEG-modified sorbitan fatty acid ester, a PEG-modified monoalkyl ether, and a PEG-modified sterol. The hydrophilic polymers may be each used alone or in a combination of two or more kinds thereof.

The molecular weight of the polyethylene glycol is not particularly limited and is 500 to 10,000 daltons, preferably 1,000 to 7,000 daltons, and more preferably 2,000 to 5,000 daltons.

Examples of the PEG-modified lipid include 1,2-distearoyl-3-phosphatidylethanolamine-polyethylene glycol such as 1,2-distearoyl-3-phosphatidylethanolamine-PEG2000 (manufactured by NOF Corporation), distearoyl glycerol PEG2000 (manufactured by NOF Corporation), or 1,2-distearoyl-3-phosphatidylethanolamine-PEG5000 (manufactured by NOF Corporation), and cholesterol-polyethylene glycol such as cholesterol-PEG600 (manufactured by Merck KGaA).

From the viewpoint of the general-purpose properties and the blood retention, the PEG-modified lipid is preferably a PEG-modified phospholipid or a PEG-modified monoalkyl ether and is more preferably a PEG-modified phospholipid.

Among the PEG-modified phospholipids, PEG-modified phosphatidylethanolamine is preferable, and diacylphosphatidylethanolamine modified with polyethylene glycol or methoxypolyethylene glycol is more preferable.

The ratio of the PEG-modified lipid in the total lipids constituting the liposome is preferably 1% to 15% by mole and more preferably 2% to 10% by mole.

The liposome according to the embodiment of the present invention contains cholesterol. The addition of cholesterol to the liposome is expected to lower the fluidity of the membrane of the liposome, for example, by filling the gaps in the membrane of the liposome.

The ratio of cholesterol in the lipid constituting the liposome is preferably 20% by mole to 50% by mole, more preferably 30% by mole to 45% by mole, and still more preferably 35% by mole to 43% by mole.

### (Topotecan or salt thereof)

The liposome according to the embodiment of the present invention encompasses topotecan or a salt thereof. The topotecan has a chemical name of (10-[(dimethylamino)methyl]-4-ethyl-4,9-dihydroxy-1H-pyrano[3',4':6,7]indolidino[1,2-b]qui noline-3,14(4H,12H)dione and is an anti-cancer agent having an inhibitory action on topoisomerase activity. In the present invention, the topotecan may be topotecan itself or may be a salt thereof, which is acceptable as a pharmaceutical product, or it may be a prodrug that liberates topotecan in vivo. In the present invention, it is preferable to use topotecan hydrochloride.

### (Ammonium sulfate in inner water phase)

The inner water phase of the liposome in the present invention contains an ammonium salt. Examples of the ammonium salt include an ammonium sulfate salt, ammonium citrate, ammonium phosphate, ammonium tartrate, ammonium succinate, ammonium fatty acid, ammonium chloride, and sucrose octasulfate as ammonium sulfate. Among them, since the retention stability of a drug can be improved by reducing the solubility of the drug in the liposome to precipitate the drug, an ammonium sulfate salt, an ammonium citrate salt, an ammonium phosphate salt, or a sucrose octasulfate as ammonium sulfate is preferable, and an ammonium sulfate salt is particularly preferable.

The molar ratio of the sulfate ions contained in the inner water phase to the molar sum of the topotecan or salt thereof contained in the anti-tumor agent according to the embodiment of the present invention is preferably 0.36 or more preferably 0.4 or more, still more preferably 0.4 or more and 1.8 or less, and particularly preferably 0.6 or more and 1.8 or less in terms of topotecan. In a case of setting the molar ratio of sulfate ions as described above, it is possible to suppress leakage of the drug from the liposome in the blood.

In addition, in the anti-tumor agent according to the embodiment of the present invention, the ratio of sulfate ions contained in the inner water phase of the liposome to sulfate ions of the entire anti-tumor agent (the inner water phase ratio of the sulfate ions) is preferably at least 80% and more preferably 90% or more. At the same time, the ratio of the topotecan or salt thereof contained in the inner water phase of the liposome to the topotecan or salt thereof of the entire anti-tumor agent (the inner water phase ratio of the drug) is preferably at least 80% and more preferably 90% or more.

The concentration of the topotecan or salt thereof in the liposome can be measured, for example, by liquid chromatography/ultraviolet-visible absorbance detection. In addition, the sulfate ions concentration in the inner water phase of the liposome can be measured, for example, by ion chromatography.

### (Production method for liposome)

A production method for the liposome according to the embodiment of the present invention is not particularly limited; however, the production method can be carried out with reference to, for example, WO2018/181963A.

### (Anti-tumor agent)

According to the present invention, an anti-tumor agent for curing cancer is provided.

The cancer in the present invention is preferably a solid cancer. Examples of the solid cancer include ovarian cancer, uterine cancer, lung cancer, Merkel cell cancer, skin cancer, breast cancer, malignant soft tissue tumor, neuroendocrine tumor, brain tumor, pharyngeal cancer, laryngeal cancer, thyroid cancer, esophageal cancer, gastric cancer, colon cancer, liver cancer, pancreatic cancer, gallbladder cancer, renal cancer, bladder cancer, prostate cancer, testis cancer, and bone tumor, where at least one selected from breast cancer, uterine cancer, ovarian cancer, lung cancer, Merkel cell cancer, neuroendocrine tumor, or brain tumor is preferable.

Examples of the ovarian cancer include serous ovarian cancer, endometrioid ovarian cancer, clear cell ovarian cancer, and mucous ovarian cancer, where serous ovarian cancer or endometrioid ovarian cancer is particularly preferable. In addition, the ovarian cancer may be ovarian cancer that is resistant to a platinum preparation (platina preparation).

Examples of the uterine cancer include cervical cancer, uterine body cancer, and uterine sarcoma, where cervical cancer or uterine sarcoma is particularly preferable.

Examples of the lung cancer include non-small cell lung cancer and small cell lung cancer, where small cell lung cancer is particularly preferable.

The resistance refers to that a tumor show resistance to an anti-cancer agent, and it includes the natural resistance to which the anti-cancer agent does not work from the beginning of curing and a condition in which an initially effective anti-cancer agent is ineffective or diminishes in effect as the curing continues. Specifically, the resistance refers to a property in which although a response to an anti-cancer agent has been exhibited in the early stage, a decrease in responsiveness is exhibited during the subsequent curing, or a suitable response to an anti-cancer agent has not been exhibited in that cancer cells have continued to proliferate during the curing using the anti-cancer agent.

It is important in cancer treatment to prolong the period during which a curing effect is obtained by changing the kinds of anti-cancer agents for tumors that have shown resistance. In addition, the kind and number of anti-cancer agents that have shown resistance may be the important information for the selection of the anti-cancer agent which is subsequently carried out.

Examples of the cancer suitable for curing according to the present invention include a cancer in which pre-curing with an anti-cancer agent has not been carried out and a cancer in which one or a plurality of kinds of pre-curing have been carried out. It is preferably a cancer which has been subjected to 3 times or less of pre-curing or a cancer which has shown resistance after curing with a platinum preparation, and it is particularly preferably ovarian cancer, cervical cancer, or small cell lung cancer, which has shown resistance to a platinum preparation and been subjected to 3 times or less of pre-curing including 3 times or less of pre-curing with the platinum preparation.

The formulation form of the anti-tumor agent according to the embodiment of the present invention is preferably a liquid medicinal preparation, and examples thereof include an injection agent.

The concentration of the topotecan or salt thereof contained in the liquid medicinal preparation according to the embodiment of the present invention is preferably 0.25 mg/mL to 5 mg/mL in terms of topotecan. It is more preferably 0.1 mg/mL to 3 mg/mL and still more preferably 0.5 mg/mL to 3 mg/mL.

The pH of the liquid medicinal preparation according to the embodiment of the present invention is preferably 5.5 to 8.5.

The liquid medicinal preparation according to the embodiment of the present invention generally may contain additives such as an emulsifying agent, a surfactant, a dissolution assisting agent, a suspending agent, an isotonizing agent, a buffering agent, a preservative, an antioxidant, a stabilizer, and an absorption promoting agent.

The isotonizing agent is not particularly limited. However, examples thereof include inorganic salts such as sodium chloride, potassium chloride, sodium hydrogen phosphate, sodium dihydrogen phosphate, and potassium dihydrogen phosphate; polyols such as glycerol, mannitol, and sorbitol; and sugars such as glucose, fructose, lactose, and sucrose.

The stabilizer is not limited to; however, examples thereof include sugars such as glycerol, mannitol, sorbitol, lactose, and sucrose.

The antioxidant is not particularly limited; however, examples thereof include ascorbic acid, uric acid, tocopherol homologues (for example, vitamin E and four isomers of tocopherol α, β, γ, and δ), cysteine, and EDTA. The stabilizer and the antioxidant can be each used alone or in a combination of two or more.

Examples of the pH adjusting agent include sodium hydroxide, citric acid, acetic acid, triethanolamine, sodium hydrogen phosphate, sodium dihydrogen phosphate, and potassium dihydrogen phosphate.

The liquid medicinal preparation according to the embodiment of the present invention may contain a pharmaceutically acceptable organic solvent (ethanol or the like), collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, gelatin, agar, diglycerin, propylene glycol, polyethylene glycol, vaseline, paraffin, stearyl alcohol, stearic acid, human serum albumin (HSA), mannitol, sorbitol, lactose, PBS, sodium chloride, sugars, an in vivo degradable polymer, a serum-free medium, and an additive acceptable as a pharmaceutical additive.

The administration method for the anti-tumor agent according to the embodiment of the present invention is preferably parenteral administration. The administration route includes routes of intravenous, intraarterial, intramuscular, intraperitoneal, subcutaneous, intraocular, and intraspinal, where a route of intravenous is preferable. Examples of the administration method include administration with a syringe or drip infusion.

The container to be filled with the liquid medicinal preparation is not particularly limited; however, it is preferably made of a material having low oxygen permeability. Examples thereof include a plastic container; a glass container; and a bag made from a laminated film.

The anti-tumor agent according to the embodiment of the present invention can also be used in combination with another active substance or a treatment method, which is useful in curing the cancer of interest. It can be used in combination with, as the treatment method, physical curing such as radiotherapy or particle beam therapy, surgical curing such as surgery, chemotherapy, molecule targeted therapy, and cancer immunotherapy. It can be used in combination with, as the other active substance, a chemotherapeutic agent that is used in chemotherapy, a molecule targeted therapeutic drug that is used in molecule targeted therapy, a cell preparation or antibody preparation which is used in cancer immunotherapy, an immune checkpoint inhibitor. Examples of the chemotherapeutic agent include an alkylating agent, an antimetabolite, an anti-tumor antibiotic, an alkaloid, a hormone therapeutic agent, a platinum complex, a topoisomerase inhibitor, and a microtubule targeted agent.

### (Dosage and dose rate)

In the anti-tumor agent according to the embodiment of the present invention, the dose rate per administration of the topotecan or salt thereof encompassed in the liposome is such that the dose rate is 0.1 mg/m² body surface area to 10 mg/m² body surface area in terms of topotecan. It is preferably 0.5 mg/m² body surface area to 5 mg/m² body surface area and more preferably 1.0 mg/m² body surface area to 3.5 mg/m² body surface area.

The anti-tumor agent according to the embodiment of the present invention is preferably repeatedly administered a plurality of times of single administration every one week to eight weeks. It is more preferably repeatedly administered a plurality of times of single administration at every one week to six weeks, it is still more preferably repeatedly administered a plurality of times of single administration at every one week to four weeks, and it is particularly preferably repeatedly administered a plurality of times of single administration at every two weeks.

The anti-tumor agent according to the embodiment of the present invention is preferably administered by infusion over 5 to 360 minutes in a single administration. 5 minutes to 240 minutes are more preferable, 10 minutes to 120 minutes are still more preferable, and 30 minutes to 120 minutes are particularly preferable.

The dose rate per administration of the topotecan or salt thereof contained in the anti-tumor agent according to the embodiment of the present invention is, for example, about 0.1 mg/m² body surface area, about 0.5 mg/m² body surface area, about 1.0 mg/m² body surface area, about 1.5 mg/m² body surface area, about 2.0 mg/m² body surface area, about 2.5 mg/m² body surface area, about 2.6 mg/m² body surface area, about 3.0 mg/m² body surface area, about 3.5 mg/m² body surface area, about 4.0 mg/m² body surface area, about 4.5 mg/m² body surface area, about 5.0 mg/m² body surface area, about 5.5 mg/m² body surface area, about 6.0 mg/m² body surface area, about 6.5 mg/m² body surface area, about 7.0 mg/m² body surface area, about 7.5 mg/m² body surface area, about 8.0 mg/m² body surface area, about 8.5 mg/m² body surface area, about 9.0 mg/m² body surface area, about 9.5 mg/m² body surface area, or about 10 mg/m² body surface area in terms of topotecan. It is preferably about 0.5 mg/m², about 1.0 mg/m² body surface area, about 1.5 mg/m² body surface area, about 2.0 mg/m² body surface area, about 2.5 mg/m² body surface area, about 2.6 mg/m² body surface area, about 3.0 mg/m² body surface area, about 3.5 mg/m² body surface area, or about 5.0 mg/m² body surface area, more preferably about 1.0 mg/m² body surface area, about 1.5 mg/m² body surface area, about 2.0 mg/m² body surface area, about 2.5 mg/m² body surface area, about 2.6 mg/m² body surface area, about 3.0 mg/m² body surface area, or, about 3.5 mg/m² body surface area, and particularly preferably about 1.0 mg/m² body surface area, about 1.5 mg/m² body surface area, about 2.0 mg/m² a body surface area, about 2.5 mg/m² body surface area, about 2.6 mg/m² body surface area, or about 3.5 mg/m² body surface area.

### Examples

The present invention will be described in more detail below according to Examples; however, the present invention is not limited to these Examples.

### <Preparation of liquid medicinal preparation containing topotecan-encompassing liposome>

With reference to WO2018/181963A, a liquid medicinal preparation (hereinafter, referred to as an A preparation) containing topotecan-encompassing liposomes having the following composition was prepared.

| | |
|---|---|
| Topotecan hydrochloride | 3.0 mg/mL (6.5 mmol) |
| DHSM (see note 1) | 10.4 mg/mL |
| DSPE-MPEG2000 (see note 2) | 3.6 mg/mL |
| Cholesterol | 3.6 mg/mL |
| Ammonium sulfate | 0.9 mg/mL (6.9 mmol) |
| Sucrose | appropriate amount |
| L-histidine | appropriate amount |
| Sodium chloride | appropriate amount |
| Water (solvent) | appropriate amount |

| | |
|---|---|
| (Note 1) Dihydrosphingomyelin prepared by chemical synthesis so that a content of a compound 1 having a long-chain alkyl group having 16 carbon atoms and a long-chain alkyl group having 18 carbon atoms is 98% or more. (Note 2) 1,2-distearoyl-sn-glycero-3-phosphoethanolamine-N-(methoxy(polyethylene glycol)-2000) | |

### <Physical property value of A preparation>

The pH of the A preparation was 7.4, and the particle diameter of the liposome contained in the A preparation was 91 nm in terms of the cumulant average particle diameter. In addition, the molar ratio of the sulfate ions contained in the inner water phase to the topotecan hydrochloride was 1.05.

### <Reference Example 1: Prediction of suitable dose in human from non-clinical test>

The A preparation was diluted with a 5% glucose solution to 0.1, 0.2, and 0.4 mg/mL, and 15 male and 15 female SD rats (Charles River Laboratories Japan, Inc.) (30 rats in total) were subjected to intravenous administration at a dose of 0.5, 1, and 2 mg/kg (respectively, 3, 6, and 12 mg/m²) 4 times (4 weeks) at an interval of once a week, where a TK group (12 males and 12 females) was set for each group.

In the group at a dose of 1 mg/kg or less, there was no death due to the A preparation during the test period. It is noted that the toxicity target organs of the A preparation were a hematopoietic system, a lymphatic system, and a digestive system.

Those in the group at a dose of 2 mg/kg were found in a dead state or sacrificed in extremis during the administration period and the day after the completion of the administration.

In the group in which the dose of the A preparation was 1 mg/kg or more, hemorrhage and decrease of neutrophils due to the dose rate-responsive myelosuppression were observed. In addition, the decrease of lymphocytes, the lymphocyte depletion in the spleen, the thymus, and the lymph nodes, and the necrosis of the gastrointestinal epithelium, as well as the hyperplasia of the intestinal crypt and the depletion of hematopoietic cells in the bone marrow were observed, which were conceived to be changes that occurred in response to the cell growth inhibitory effect of the A preparation. All of these changes showed recoverability after a recovery period of 4 weeks. On the other hand, in the group at a dose of 0.5 mg/kg, the change due to the A preparation was not observed.

Based on the above results, the maximum tolerated dose rate in a rat was set to 1 mg/kg (6 mg/m²) at which no death is observed, and the initial dose rate in a human was set to 1.0 mg/m², which is about 1/10 of the maximum tolerated dose rate.

### <Reference Example 2: Relationship between toxicity and blood kinetics (toxicokinetics)>

In a case where 1 mg/kg, which is the maximum tolerated dose rate of the A preparation obtained in Reference Example 1, was intravenously administered to a rat, the total topotecan exposure in the blood plasma was 28,400 ng/mL (Cmax) and 563,000 h·ng/mL (AUC₀₋₉₆), and the exposure in a case of administration at 0.5 mg/kg, at which no toxicity was observed, was 11,000 ng/mL (Cmax) and 491,000 h·ng/mL (AUC₀₋₉₆).

### <Reference Example 3: Temporal change of toxicity profile after administration>

The A preparation was diluted with a 5% glucose solution to 0.05, 0.1, and 0.2 mg/mL, and 6 male SD rats (Charles River Laboratories Japan, Inc.) were each subjected to single intravenous administration at a dose of 0.25, 0.5, and 1 mg/kg (respectively, 1.5, 3, and 6 mg/m²). Blood was collected at 1, 3, 5, 7, 10, 14, and 21 days after the administration, and a hematological test, a feeding amount measurement, and a body weight measurement were carried out. It is noted that no dead case or moribund case was observed at all dose rates.

In the group at a dose of 1 mg/kg, the low values of the erythroid system and the leukocyte system were observed until 14 days after the administration, which tended to recover from 10 days after the administration. The suppression of the body weight increase was observed until 5 days after the administration, which was recovered 10 days after the administration.

In the group at a dose of 0.5 mg/kg or less, any change was slight as compared with the group at a dose of 1 mg/kg, and the recovery was rapid.

From the above, it was found that in a case where a rat is subject to a single intravenous administration of 1 mg/kg, changes in the hematopoietic system, the lymphatic system, and the digestive system, which are the toxicity target organs of the A preparation, are recovered on the 14th day of the administration.

### <Reference Example 4: Prediction of suitable administration schedule in human from non-clinical test>

Based on the rat toxicokinetics (TK) data obtained in Reference Example 2, the total topotecan exposure of the A preparation in a human was calculated using population pharmacokinetics (PPK).

As a result of calculating the clearance of the total topotecan per body weight based on the PPK model, it was 1.86 mL/h/kg (rat). As a result of fitting the clearance in a human with exponentiation with the body weight as a base according to the CL = a*BW^{b}, the clearance in a human was calculated to be 113 mL/h or 1.61 mL/h/kg in terms of body weight in a patient having a body weight of 70 kg. The predicted value of the clearance was 100 times lower than 18.6 L/hr which is a clearance in a case where topotecan is intravenously administered (reference document: Mould et al. 2002, Ait-Oudhia, Mager and Straubinger 2014).

Using the clearance in a human calculated in the PPK model, a simulation was carried out in a case where the A preparation was administered to a human once every two weeks at a dose from 1 to 12 mg/m², and as a result, t_{1/2} of the total topotecan was 18.5 hours (90% reliability interval: 13.2 to 24.6 hours), which was 9 times longer than the clearance in a case where topotecan is intravenously administered (reference document: HYCAMTIN, attached document). There was no accumulation and a steady state was observed in a case of being administered once every two weeks between 1 and 12 mg/m².

From the above results, it was predicted to be suitable to administer the A preparation once every two weeks due to the decrease in clearance due to liposomization and the extension of t_{1/2}. In addition, in the rat test of Reference Example 3, it was confirmed that the change due to the toxicity is recovered 2 weeks after the administration, and from this viewpoint as well, it was predicted that the administration once every two weeks is suitable.

### <Example: Administration test>

Based on the dose predicted from Reference Example 1, the A preparation was used in the curing shown in Examples 1 and 2 below. It is noted that the curing was carried out at Honor Health Research Institute located in Scottsdale, Arizona, USA, Sarah Cannon Research Institute located in Denver, Colorado, USA, University of Texas, M.D. Anderson Cancer Center, located in Houston, Texas, USA, and Dana Farber Cancer Institute located in Boston, Massachusetts, USA.

The drug administration cycle of administering the A preparation to a cancer patient once every two weeks was repeated. Specifically, 28 days were set as one cycle, the A preparation was administered on the 1st day and the 15th day, and the cycle consisting of these 28 days was repeated.

The curing effect was determined according to the following criteria.

The subject to be evaluated was checked by image diagnosis by computed tomography (CT) or magnetic resonance imaging (MRI), and the evaluation was made according to the following criteria.
Complete response (CR): A state in which the tumor is completely disappeared.
Partial response (PR): A state in which the size of the tumor is reduced by 30% or more as compared with the sum of the sizes of the tumor before drug administration.
Progressive disease (PD): A state in which the size of the tumor is increased by 20% or more and the absolute value thereof is increased by 5 mm or more as compared with the sum of the sizes of the smallest tumor during the progress, or a state in which a new lesion has appeared.
Stable disease (SD): A state in which there is no reduction corresponding to PR and no increase corresponding to PD as compared with the sum of the sizes of the smallest tumors during the progress.

It is noted that since the purpose of chemotherapy for solid cancer is to relieve symptoms and prolong life, it is determined to be effective as a drug effect even in a case where the curing effect is SD.

### 1. Ovarian cancer patient 1

In an ovarian cancer patient 1 to which the A preparation was administered at 2.0 mg/m² in terms of topotecan per administration, SD was confirmed, and the curing was continued for about 24 weeks.

This patient had received, as pre-curing, drug therapy with a combined use of carboplatin, paclitaxel, and avelumab, a combined use of carboplatin and nab-paclitaxel, and a combined use of liposomal doxorubicin and bevacizumab. The patient was 76 years old and was female.

### 2. Ovarian cancer patient 2

In an ovarian cancer patient 2 to which the A preparation was administered at 2.0 mg/m² in terms of topotecan per administration, SD was confirmed, and the curing was continued for about 24 weeks.

This patient had received, as pre-curing, drug therapy with a combined use of carboplatin and paclitaxel, a combined use of carboplatin, gemcitabine, and bevacizumab, a single agent of bevacizumab, and a single agent of topotecan. The patient was 79 years old and was female.

### 3. Ovarian cancer patient 3

In an ovarian cancer patient 3 in which the administration of the A preparation was started at 3.5 mg/m² in terms of topotecan per administration and the amount thereof was decreased to 2.63 mg/m² from 4 weeks after the start, PR was confirmed, and the curing was continued for about 32 weeks.

This patient had received, as pre-curing, drug therapy with a combined use of carboplatin and paclitaxel. The patient was 71 years old and was female.

The curing effect on the ovarian cancer patient 3 was confirmed according to image diagnosis by CT. The results are shown in Fig. 1. a of Fig. 1 is a baseline image before the administration of the A preparation. b of Fig. 1 is a CT image in a case where PR was confirmed 8 weeks after the administration. c of Fig. 1 is a CT image in a case where it is confirmed that PR is continued after 16 weeks have passed from the administration. As shown in Fig. 1, in a case where the A preparation is administered at the dosage and dose rate according to the embodiment of the present invention, a very excellent effect is exhibited in the curing of the ovarian cancer.

### 4. Ovarian cancer patient 4

In an ovarian cancer patient 4 to which the A preparation was administered at 3.5 mg/m² in terms of topotecan per administration, SD was confirmed, and the curing was continued for about 44 weeks.

This patient had received, as pre-curing, drug therapy with a combined use of carboplatin and paclitaxel, a combined use of carboplatin and liposomal doxorubicin, a single agent of paclitaxel, a single agent of carboplatin, and the like. The patient was 59 years old and was female.

### 5. Uterine sarcoma patient

In a uterine sarcoma patient to which the A preparation was administered at 2.5 mg/m² in terms of topotecan per administration, SD was confirmed, and the curing was continued for about 16 weeks.

This patient had received, as pre-curing, drug therapy with a combined use of carboplatin and paclitaxel and a single agent of zeluvalimab. The patient was 70 years old and was female.

### 6. Uterine leiomyosarcoma patient

In a uterine leiomyosarcoma patient to which the A preparation was administered at 2.5 mg/m² in terms of topotecan per administration, SD was confirmed, and the curing was continued for about 22 weeks.

This patient had received, as pre-curing, drug therapy with a combined use of gemcitabine and docetaxel, a combined use of doxorubicin and dacarbazine, and a single agent of dacarbazine. The patient was 44 years old and was female.

### 7. Cervical cancer patient

In a cervical cancer patient in which the administration of the A preparation was started at 3.0 mg/m² in terms of topotecan per administration and the amount thereof was decreased to 2.5 mg/m² from 4 weeks after the start, PR was confirmed, and the curing was continued for about 51 weeks.

This patient had received, as pre-curing, drug therapy with a combined use of carboplatin, paclitaxel, and bevacizumab and a combined use of lucitanib and nivolumab. The patient was 73 years old and was female.

The curing effect on the cervical cancer patient was confirmed according to image diagnosis by CT. The results are shown in Fig. 2. a and b of Fig.2 are baseline images before the administration of the A preparation. c and d of Fig. 2 are CT images in a case where PR is confirmed after 10 months have passed from the administration and PR is confirmed to be continued after 2 months further passed. It is noted that a and c of Fig. 2, and b and d of Fig. 2 are respectively paired, which shows that the size of the tumor is reduced by the administration of the A preparation. Therefore, in a case where the A preparation is administered at the dosage and dose rate according to the embodiment of the present invention, a very excellent effect is exhibited in the curing of the cervical cancer.

### 8. Ovarian cancer patient 5

In an ovarian cancer patient in which the administration of the A preparation was started at 2.5 mg/m² in terms of topotecan per administration and the amount thereof was decreased to 1.875 mg/m² from 4 weeks after the start, SD was confirmed, and the curing was continued for about 38 weeks.

This patient had received, as pre-curing, drug therapy with a combined use of carboplatin and paclitaxel, a bevacizumab maintenance therapy, a combined use of carboplatin and liposomal doxorubicin, a single agent of IMGN 853, a single agent of olaparib, a single agent of PREXASERTIB, a combined use of DKN-01 and paclitaxel, a combined use of carboplatin and cyclophosphamide, and a single agent of oral cyclophosphamide. The patient was 76 years old and was female.

### 9. Ovarian cancer patient 6

In an ovarian cancer patient in which the administration of the A preparation was started at 2.0 mg/m² in terms of topotecan per administration and the amount thereof was decreased to 1.5 mg/m² from 6 weeks after the start, SD was confirmed, and the curing was continued for about 12 weeks.

This patient had received, as pre-curing, drug therapy with a combined use of carboplatin and paclitaxel, a combined use of liposomal doxorubicin and bevacizumab, and a combined use of paclitaxel and bevacizumab. The patient was 46 years old and was female.

### 10. Ovarian cancer patient 7

In an ovarian cancer patient to which the A preparation was administered at 2.0 mg/m² in terms of topotecan per administration, SD was confirmed, and the curing was continued for about 11 weeks.

This patient had received, as pre-curing, drug therapy with a combined use of carboplatin, paclitaxel, and bevacizumab and a single agent of letrozole. The patient was 51 years old and was female.

### 11. Ovarian cancer patient 8

In an ovarian cancer patient to which the A preparation was administered at 2.0 mg/m² in terms of topotecan per administration, SD was confirmed, and the curing was continued for about 10 weeks.

This patient had received, as pre-curing, drug therapy with a combined use of carboplatin and paclitaxel. The patient was 59 years old and was female.

An excellent anti-tumor effect was obtained from the anti-tumor agent according to the embodiment of the present invention. Specifically, the effect is an effect of reducing the volume of the cancer or an effect of not changing the size of the tumor. From these results, the anti-tumor agent according to the embodiment of the present invention is expected to prolong the progression-free survival time and the overall survival time of patients, and it has a very useful effect from the viewpoint of improving the QOL of patients.

The anti-tumor agent according to the embodiment of the present invention is useful since it exhibits an excellent anti-tumor effect.

## Claims

1. An anti-tumor agent for curing cancer, the anti-tumor agent comprising:
a liposome which has an inner water phase; and
an aqueous solution which is an outer water phase and disperses the liposome,
wherein the liposome encompasses topotecan or a salt thereof,
a lipid constituting the liposome contains a lipid modified with polyethylene glycol, dihydrosphingomyelin, and cholesterol,
the inner water phase contains an ammonium salt, and
the topotecan or the salt thereof encompassed in the liposome is administered at a dose rate of 0.1 mg/m² body surface area to 10 mg/m² body surface area, in terms of topotecan per administration.

2. The anti-tumor agent according to claim 1,
wherein the dihydrosphingomyelin is dihydrosphingomyelin having an alkyl group having 16 carbon atoms and an alkyl group having 18 carbon atoms.

3. The anti-tumor agent according to claim 1 or 2,
wherein the ammonium salt contained in the inner water phase of the liposome contained in the anti-tumor agent is ammonium sulfate, and
a molar ratio of sulfate ions contained in the inner water phase to the molar sum of the topotecan or the salt thereof contained in the anti-tumor agent is 0.36 or more in terms of topotecan.

4. The anti-tumor agent according to any one of claims 1 to 3,
wherein a plurality of times of single administration is repeated every one week to eight weeks.

5. The anti-tumor agent according to any one of claims 1 to 4,
wherein the cancer is a solid cancer.

6. The anti-tumor agent according to claim 5,
wherein the solid cancer is at least one selected from breast cancer, uterine cancer, ovarian cancer, lung cancer, Merkel cell cancer, neuroendocrine tumor, or brain tumor.

7. The anti-tumor agent according to any one of claims 1 to 6,
wherein the lipid modified with polyethylene glycol is diacylphosphatidylethanolamine modified with polyethylene glycol or methoxypolyethylene glycol.

8. The anti-tumor agent according to any one of claims 1 to 7,
wherein a formulation ratio of the cholesterol to a total of the lipid constituting the liposome is 35% to 43% by mole.

9. The anti-tumor agent according to any one of claims 1 to 8,
wherein a pH of the outer water phase is 5.5 to 8.5.

10. The anti-tumor agent according to any one of claims 1 to 9,
wherein an administration route is an intravenous administration.

11. The anti-tumor agent according to any one of claims 1 to 10,
wherein the anti-tumor agent is administered by infusion for 5 minutes to 360 minutes in a single administration.
